# FASCICULE DE BREVET EUROPEEN

(11) **EP 4 394 559 B1**
(45) Date de publication et mention de la délivrance du brevet: **30.09.2026**
(21) Numéro de dépôt: 23220422.2
(22) Date de dépôt: 28.12.2023
(51) Int. Cl.: G06F 3/01, A61F 2/72, A61F 4/00

(54) **INTERFACE NEURONALE DIRECTE POUR PILOTER LE DÉPLACEMENT D'UN EFFECTEUR**
DIREKTE NEURONALE SCHNITTSTELLE ZUR STEUERUNG DER BEWEGUNG EINES EFFEKTORS
DIRECT NEURAL INTERFACE TO CONTROL MOVEMENT OF AN EFFECTOR

(30) Priorité: 29.12.2022 FR 2214664
(43) Date de publication de la demande: 03.07.2024
(73) Titulaire: Commissariat à l'Energie Atomique et aux Energies Alternatives, 75015 Paris (FR)
(72) Inventeur: MARTEL, Felix, 38054 Grenoble cedex 09 (FR); SID-AHMED, Hafid, 38054 Grenoble cedex 09 (FR); AKSENOVA, Tetiana, 38054 Grenoble cedex 09 (FR)
(74) Mandataire: INNOV-GROUP

(56) Documents cités:
- US-A1- 2003 093 129
- US-A1- 2016 224 891

## Description

### DOMAINE TECHNIQUE

L'invention concerne une interface neuronale directe, usuellement désignée par l'acronyme anglosaxon BCI, signifiant Brain Computer Interface, pour permettre la commande d'un effecteur automatisé, par exemple un robot ou un exosquelette, par des signaux électrophysiologiques corticaux.

### ART ANTERIEUR

Le domaine des interfaces neuronales directes est en plein développement est apparaît comme une solution séduisante pour permettre à des personnes handicapées de commander des effecteurs par la pensée. Il s'agit d'enregistrer, généralement à l'aide d'électrodes corticales, des signaux électrophysiologiques. Ces derniers sont traités par des algorithmes permettant d'extraire un signal de commande, pour commander des effecteurs. Le signal de commande peut permettre le pilotage d'un effecteur de type exosquelette, orthèse ordinateur ou robot, pour fournir une assistance à l'utilisateur. Les algorithmes mis en œuvre permettent une traduction d'une instruction donnée par l'utilisateur, cette instruction étant captée, par les électrodes, sous la forme de signaux dits électrophysiologiques, car représentatifs de l'activité électrique des neurones. Cette activité électrique est généralement mesurée au niveau du cortex, au moyen d'électrodes corticales disposées dans la boîte crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en œuvre sont généralement basés sur un modèle prédictif. Le modèle prédictif utilise des données d'entrée, obtenues par un prétraitement des signaux électrophysiologiques enregistrés. Un exemple de calibration d'un modèle prédictif est décrit dans US11497429. Le modèle prédictif permet un traitement des données d'entrée pour établir un déplacement prédit, permettant d'effectuer un déplacement de l'effecteur correspondant aux signaux électrophysiologiques détectés.

D'autres algorithmes adaptés à la commande d'une interface BCI sont décrits dans US20030093129 ou dans US20160224891.

Cependant, le recours à un modèle prédictif peut s'accompagner d'une certaine imprécision, lorsque l'utilisateur cherche à déplacer l'effecteur vers une position cible prédéterminée. L'invention permet de résoudre ce problème.

### EXPOSE DE L'INVENTION

Un premier objet de l'invention est un procédé de commande du déplacement, dans un espace, d'un effecteur, par une interface neuronale directe, le déplacement tendant à rapprocher l'effecteur d'une position cible, comportant les étapes suivantes :
- a) acquisition de signaux électrophysiologiques produits dans le cortex d'un individu, à un instant de mesure, l'effecteur occupant une position, dans l'espace, audit instant de mesure ;
- b) traitement des signaux électrophysiologiques, pour former des données d'entrée ;
- c) traitement des données d'entrée, par un modèle prédictif mis en œuvre par l'interface neuronale, pour définir un déplacement de l'effecteur à l'instant de mesure;
- d) détermination d'un déplacement corrigé de l'effecteur, à partir du déplacement défini par le modèle prédictif lors de l'étape c) ;
- e) commande du déplacement de l'effecteur dans l'espace, par l'interface neuronale directe, à partir du déplacement corrigé résultant de l'étape d) ;
- f) réitération des étapes a) à e), en incrémentant l'instant de mesure, jusqu'à l'atteinte d'un critère d'arrêt des itérations ;
le procédé comportant, lors de chaque étape d) suivant au moins une première itération des étapes a) à e) :
- di) estimation de la position cible, à l'instant de mesure, à partir d'au moins :
   - une position de l'effecteur à l'instant de mesure, ainsi qu'à au moins un instant précédent l'instant de mesure ;
   - un déplacement défini par le modèle prédictif à l'instant de mesure ainsi qu'à au moins l'instant précédent l'instant de mesure ;
- dii) à partir de la position cible estimée lors de la sous-étape di), détermination d'un déplacement vers la cible estimée;
- diii) prise en compte du déplacement vers la cible estimée, résultant de la sous-étape dii), pour établir le déplacement corrigé à l'instant de mesure.

La sous-étape di) peut comporter :
- calcul d'une densité de probabilité de cible, correspondant à une densité de probabilité que chaque point de l'espace corresponde à la position cible ;
- détermination du point de l'espace maximisant la densité de probabilité de cible, ou une grandeur comportant la densité de probabilité de cible, le point ainsi déterminé correspondant à la position cible estimée.

Le calcul de la densité de probabilité de cible peut prend en compte :
- plusieurs positions de l'effecteur à des instants précédent l'instant de mesure ;
- plusieurs déplacements définis par le modèle prédictif à des instants précédents l'instant de mesure.

Le calcul de la densité de probabilité de cible peut être établi à partir d'une combinaison, par exemple linéaire,
- d'une première densité de probabilité conditionnelle conditionnée par
   - des positions de l'effecteur à des instants précédent l'instant de mesure;
   - des déplacements définis par le modèle prédictif à des instants précédent l'instant de mesure ;
- et d'une deuxième densité de probabilité conditionnelle conditionnée par:
   - la position de l'effecteur à l'instant de mesure ;
   - le déplacement défini par le modèle prédictif à l'instant de mesure.

La première densité de probabilité conditionnelle peut être pondérée par un facteur d'oubli. La sous-étape di) peut comporter une détermination du point maximisant une grandeur comportant la densité de probabilité cible, la grandeur correspondant à la densité de probabilité cible diminuée :
- d'une première distance, pondérée par un premier facteur de régularisation, entre chaque point et la position cible estimée à un instant de mesure précédent ;
- et/ou d'une deuxième distance, pondérée par un deuxième facteur de régularisation entre chaque point et la position de l'effecteur à l'instant de mesure.

Lors de la sous-étape dii), le déplacement vers la cible estimée peut être établi de façon que l'interface déplace l'effecteur de la position à l'instant de mesure vers la position cible estimée à l'instant de mesure.

Lors de la sous-étape diii), le déplacement corrigé peut être une combinaison, par exemple une combinaison linéaire, du déplacement défini par le modèle prédictif lors de l'étape b) et du déplacement vers la cible estimée résultant de la sous-étape dii). La combinaison linéaire peut mettre en œuvre :
- un premier facteur de pondération, appliqué au déplacement défini par le modèle prédictif ;
- un deuxième facteur de pondération, appliqué au déplacement vers la cible estimée.

Un deuxième objet de l'invention est une interface neuronale directe, comportant :
- des capteurs, aptes à détecter des signaux électrophysiologiques représentatifs d'une activité corticale ;
- un effecteur, configuré pour être actionné par un signal de commande généré par l'interface neuronale directe ;
- un processeur programmé pour déterminer un déplacement de l'effecteur en fonction de données d'entrée résultant des signaux électrophysiologiques détectés ;
le processeur étant configuré pour mettre en œuvre les étapes c) à f) d'un procédé selon le premier objet de l'invention, en différents instants de mesure.

L'invention sera mieux comprise à la lecture de l'exposé des exemples de réalisation présentés, dans la suite de la description, en lien avec les figures listées ci-dessous.

### FIGURES

La figure 1 schématise les principaux composants d'une interface neuronale directe.
La figure 2 représente un déplacement d'un effecteur dans un environnement 3D.
La figure 3 illustre un vecteur correspondant à un déplacement prédit par un modèle prédictif, un déplacement désiré, entre la position de l'effecteur et la position de la cible, ainsi que l'erreur angulaire entre les deux vecteurs.
La figure 4 montre les principales étapes d'un procédé mis en œuvre par une interface neuronale selon l'invention.
Les figures 5A et 5B montrent l'évolution de distances entre l'effecteur et la cible, ainsi qu'entre la cible estimée et la cible réelle, en fonction du temps, en considérant deux modèles de bruits différents. Sur les figures 5A et 5B, l'effecteur est déplacé vers la cible sans prendre en compte les positions estimées de la cible au cours du temps.
Les figures 6A et 6B montrent l'évolution de précisions angulaires en fonction du temps, en considérant deux modèles de bruits différents.
Les figures 7A à 7D montrent l'influence de la pondération du déplacement de l'effecteur entre le déplacement prédit par l'interface et un déplacement corrigé, en mettant en œuvre l'invention, en fonction d'une estimation de la position de la cible.
La figure 8 illustre une mise en œuvre de l'invention en 3D.

### EXPOSE DE MODES DE REALISATION PARTICULIERS

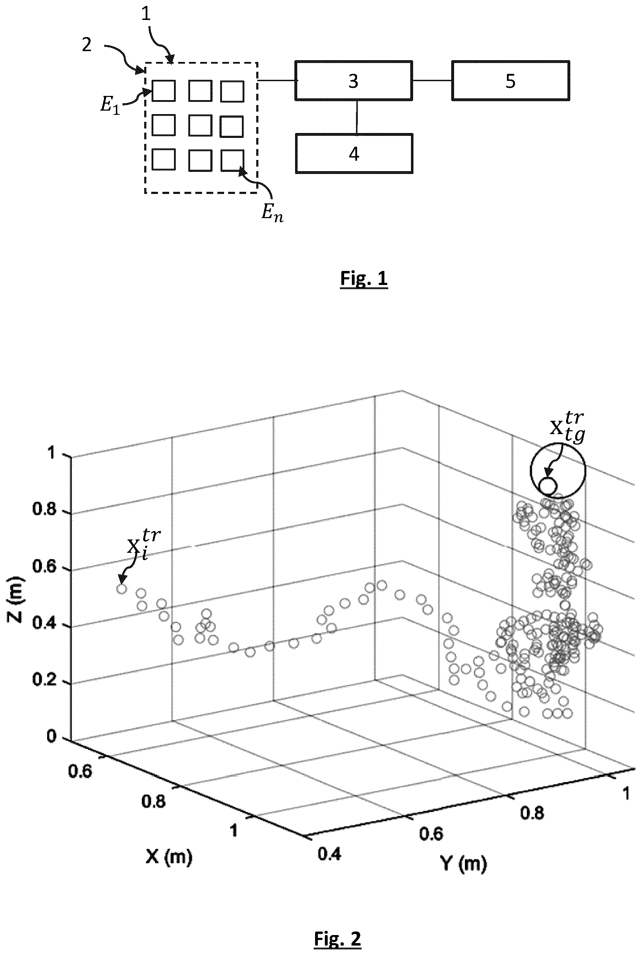

La figure 1 représente les principaux éléments d'une interface neuronale directe 1 selon l'invention. Il s'agit d'un dispositif comportant des capteurs 2, permettant l'acquisition de signaux électrophysiologiques représentatifs d'une activité neuronale. Les capteurs 2 sont par exemple des électrodes corticales E₁....Eₙ. Les capteurs 2 sont reliés à un processeur 3, par une liaison filaire ou sans fil. Le processeur 3 est apte à exécuter des instructions codées dans une mémoire 4. Ces instructions comprennent notamment les algorithmes permettant de générer un signal de commande destiné à piloter un effecteur 5.

L'effecteur 5 est un actionneur apte à effectuer une action sous l'effet du signal de commande qui lui est adressé. Il peut s'agir d'un exosquelette, d'un robot, ou d'un ordinateur. L'effecteur est alors commandé par les signaux électrophysiologiques acquis par les électrodes corticales 2, via un algorithme codé dans la mémoire 4 et mis en œuvre par le processeur 3. Plus précisément, l'objectif du dispositif est de permettre un déplacement de l'effecteur, dans un environnement, jusqu'à une position cible.

L'environnement peut être un environnement réel, l'effecteur étant par exemple un bras robotique, une orthèse robotisée, un gant ou un outil porté par la main de l'utilisateur. L'objectif est de déplacer l'effecteur jusqu'à la position cible, qui est une position que l'utilisateur souhaite atteindre. L'environnement peut être virtuel, l'effecteur étant par exemple une souris d'ordinateur. L'utilisateur peut alors piloter un curseur vers une position déterminée sur un écran, de façon à activer une commande. La position cible est la position du curseur que l'utilisateur souhaite atteindre.

Le dispositif 1 est une interface neuronale directe, dans le sens où il permet le contrôle de l'effecteur 5 par le les signaux électrophysiologiques mesurés par les capteurs 2. Les signaux électrophysiologiques peuvent être acquis par des électrodes corticales placées au contact du cortex sensori-moteur, des capteurs de magnétoencéphalographie ou des électrodes d'électroencéphalographie.

Le signal de commande est formé par le processeur 3 à partir des signaux électrophysiologiques, L'algorithme met en œuvre un modèle prédictif, générant un signal de sortie, permettant le contrôle de l'effecteur 5. Le modèle prédictif peut être déterminé comme décrit dans le brevet US11497429. Plus précisément, le signal de sortie permet de déplacer l'effecteur 5 jusqu'à la position cible selon un déplacement prédit par le modèle prédictif.

L'espace peut être discrétisé en une pluralité de points. La figure 2 illustre un déplacement progressif d'un effecteur entre une position initiale ${x}_{i}^{tr}$ et une position cible ${x}_{tg}^{tr}$. L'exposant *tr* désigne l'essai réalisé. Le déplacement est effectué de façon saccadée, en fonction de la fréquence d'échantillonnage de l'interface, cette dernière pouvant être de 10 Hz. La fréquence d'échantillonnage est de préférence suffisamment rapide pour ne pas paraître trop saccadée pour l'utilisateur, et pour limiter le temps de réaction du système quand l'utilisateur change d'intention. A chaque instant de mesure s, l'effecteur occupe une position ponctuelle ${x}_{c}^{s}$. ${x}_{c}^{s}$ est un point représenté par un vecteur dont la dimension correspond à la dimension de l'espace dans lequel l'effecteur évolue. Dans un premier mode de réalisation, on considère un espace à 2 dimensions. Chaque vecteur est défini par une coordonnée x et une coordonnée y.

A chaque instant s, le modèle prédictif de l'interface génère un déplacement prédit ${x}_{p}^{s}$, qui correspond au déplacement correspondant aux signaux électrophysiologiques détectés par l'interface. ${x}_{p}^{s} = {x}_{c}^{s + 1} - {x}_{c}^{s}$ Le déplacement ${x}_{p}^{s}$ correspond au déplacement de l'effecteur, tel que prédit par le modèle prédictif, entre deux instants successifs s, *s+1,* en fonction des signaux électrophysiologiques détectés à l'instant s.

A chaque instant s, on peut déterminer un vecteur ${x}_{d}^{s}$, qui correspond à un déplacement désiré, tel que : ${x}_{d}^{s} = {x}_{tg}^{tr} - {x}_{c}^{s}$

Cependant, la position ${x}_{tg}^{tr}$, bien qu'elle soit connue par l'utilisateur, n'est pas connue par l'interface. Il est donc nécessaire de l'estimer, ce qui est décrit par la suite, de façon à pouvoir déterminer ${x}_{d}^{s}$. ${x}_{d}^{s}$ correspond à un vecteur de déplacement idéal, qui serait suivi par l'effecteur sans les imperfections de l'interface. On se place dans une hypothèse selon laquelle l'utilisateur a l'intention de déplacer l'effecteur vers la cible selon un déplacement en ligne droite.

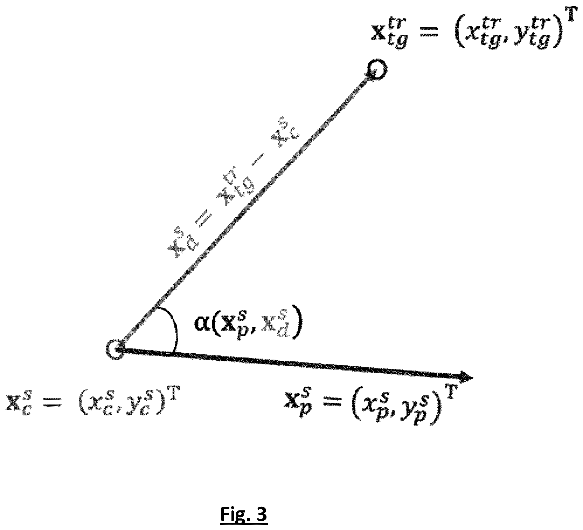

Sur la figure 3, on a représenté les vecteurs ${x}_{d}^{s}$ et ${x}_{p}^{s}$. L'angle entre les deux vecteurs ${x}_{d}^{s}$ et ${x}_{p}^{s}$ correspond à une erreur angulaire, notée $α \left({x}_{p}^{s}, {x}_{d}^{s}\right)$.

Un élément important de l'invention est l'estimation, par l'interface, en chaque instant de mesure s, de la position de la cible $\hat{{x}_{tg}^{s}}$*.* L'estimation de la position de la cible est obtenue en maximisant une probabilité, dite probabilité de cible, définie en tout point de l'espace, et correspondant à la probabilité que chaque point de l'espace corresponde à la cible ${x}_{tg}^{tr}$.

La probabilité de cible est une probabilité conditionnelle, définie en plusieurs points de l'espace, et de préférence en tout point de l'espace. En faisant l'hypothèse que l'utilisateur souhaite commander un déplacement de l'effecteur en ligne droite vers la position cible, donc cherche à réduire l'erreur angulaire, on peut estimer que pour chaque point de l'espace, représenté par un vecteur x, de coordonnées *(x, y) ;* l'erreur angulaire $α \left({x}_{p}^{s},x-{x}_{c}^{s}\right)$ est une variable suivant une densité de probabilité gaussienne, de variance σ². $P \left(x={x}_{tg}^{tr}\left|{x}_{c}^{s}, {x}_{p}^{s}\right.\right) = \frac{1}{\sqrt{2 {πσ}^{2}}} {e}^{- \frac{α {\left({x}_{p}^{s},x-{x}_{c}^{s}\right)}^{2}}{2 {σ}^{2}}}$

La densité de probabilité cible $P \left(x={x}_{tg}^{tr}\left|{x}_{c}^{s}, {x}_{p}^{s}\right.\right)$ est estimée à chaque instant de mesure s.

La variance σ² peut être estimée comme décrit par la suite.

Avantageusement, la densité de probabilité de cible peut être estimée en prenant en compte les positions de l'effecteur ${x}_{c}^{s = 1} … {x}_{c}^{s}$ et les déplacements prédits par le modèle prédictif ${x}_{p}^{s = 1} … {x}_{p}^{s}$ à des instants précédents l'instant de mesure. Ainsi, la densité de probabilité de cible est telle que : $P \left(x={x}_{tg}^{tr}\left|{x}_{c}^{1}… {x}_{c}^{s},{x}_{p}^{1}… {x}_{p}^{s}\right.\right) = \frac{1}{s} {\sum }_{i = 1}^{s} P \left(x={x}_{tg}^{tr}\left|{x}_{c}^{i}, {x}_{p}^{i}\right.\right)$

Pour un point de position x , la densité de probabilité selon l'expression (4) correspond à un moyennage des probabilités cibles établies au cours du temps pour ledit point.

A chaque instant s, la position de la cible est considérée comme celle maximisant la densité de probabilité de cible. $\hat{{x}_{tg}^{s}} = arg {max}_{x} \left[P\left(x={x}_{tg}^{tr}\left|{x}_{c}^{1}… {x}_{c}^{s},{x}_{p}^{1}… {x}_{p}^{s}\right.\right)\right]$

A chaque instant s, l'estimation de la position de la cible $\hat{{x}_{tg}^{s}}$ est utilisée pour commander le déplacement de l'effecteur. On établit un déplacement vers la cible estimée, ${x}_{p , MPT}^{s}$*,* tel que :${x}_{p , MPT}^{s} = \hat{{x}_{tg}^{s}} - {x}_{c}^{s}$
${x}_{p , MPT}^{s}$ est le vecteur entre la position courante ${x}_{c}^{s}$ et la position estimée de la cible $\hat{{x}_{tg}^{s}}$*.* Il s'agit du vecteur de déplacement vers la cible estimée.
${x}_{d}^{s}$ est le vecteur entre la position courante ${x}_{c}^{s}$ et la position réelle de la cible ${x}_{tg}^{s}$*.* Il s'agit du vecteur de déplacement désiré.

La position de l'effecteur à l'instant ${x}_{c}^{s + 1}$ peut être estimée par l'expression : ${x}_{c}^{s + 1} = β \left(k\frac{{x}_{p}^{s}}{\left‖{x}_{p}^{s}\right‖}+\left(1-k\right)\frac{{x}_{p , MPT}^{s}}{\left‖{x}_{p , MPT}^{s}\right‖}\right) + {x}_{c}^{s}$

Où k est un premier facteur de pondération et 1 - k est un deuxième facteur de pondération. Dans cet exemple, 0≤k≤1. *β* est un réel positif, correspondant à une distance de déplacement préalablement déterminée. Choisir k = 1 revient à ne pas prendre en compte la position estimée de la cible, ce qui revient à un déplacement selon l'art antérieur. Choisir k = 0 revient à ne prendre en compte que l'estimation de la cible dans la commande du déplacement.

Le premier facteur de pondération k et le deuxième facteur de pondération 1 - k permettent de doser les contributions :
- du déplacement prédit par le modèle prédictif de l'interface ;
- du déplacement vers la position estimée de la cible.

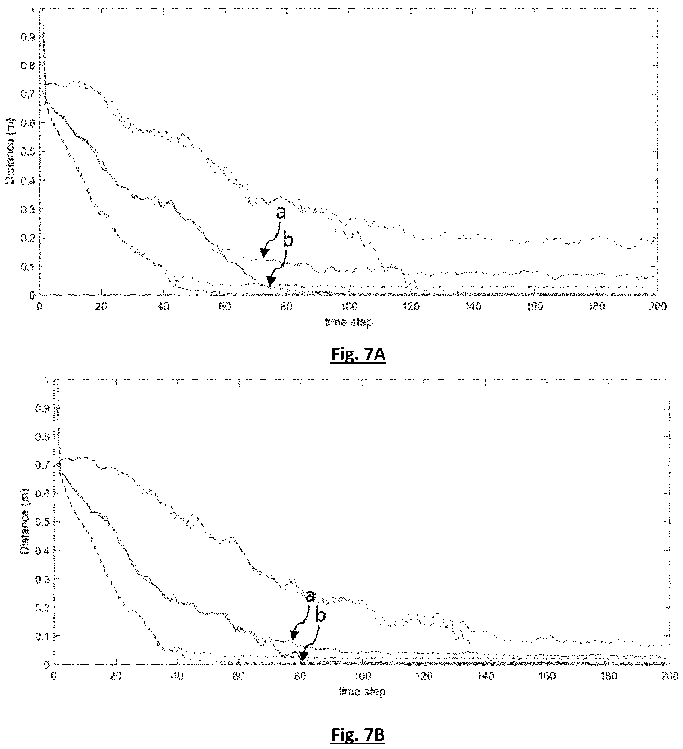

L'influence de ces facteurs de pondération est discutée en lien avec les figures 7A à 7D.

Le déplacement ${x}_{c}^{s + 1} - {x}_{c}^{s}$ correspond à un déplacement corrigé, prenant en compte à la fois le déplacement prédit par le modèle prédictif, et le déplacement vers la position estimée de la cible. Il s'agit d'un aspect clef de l'invention.

Ainsi, le déplacement de l'effecteur est en partie défini par le modèle prédictif, comme dans l'art antérieur, et en partie défini en fonction de la position estimée de la cible $\hat{{x}_{tg}^{s}}$*.* La prise en compte de la position estimée de la cible, dans la détermination du déplacement de l'effecteur, est un aspect important de l'invention.

Selon une variante, la densité de probabilité de cible est telle que: $P \left(x={x}_{tg}^{tr}\left|{x}_{c}^{1}…{x}_{c}^{s},{x}_{p}^{1}…{x}_{p}^{s}\right.\right) = \frac{\left(s-1\right) . μ . P \left(x={x}_{tg}\left|{x}_{c}^{1}…{x}_{c}^{s - 1},{x}_{p}^{1}…{x}_{p}^{s - 1}\right.\right) + P \left(x={x}_{tg}\left|{x}_{c}^{s}, {x}_{p}^{s}\right.\right)}{s}$ où *µ* est un réel positif, correspondant à un facteur d'oubli. *µ* permet de doser la prise en compte des instants antérieurs par rapport à l'instant courant s. *µ* est généralement compris entre 0 et 1. *µ* permet de moduler l'impact des instants anciens dans la prise de décision.

Selon cette variante, la densité de probabilité de cible est établie à partir d'une combinaison linéaire :
- d'une première densité de probabilité conditionnelle $P \left(x={x}_{tg}\left|{x}_{c}^{1}…{x}_{c}^{s - 1},{x}_{p}^{1}…{x}_{p}^{s - 1}\right.\right)$ , pondérée par le facteur d'oubli *µ*, et conditionnée par les positions de l'effecteur en des instants précédent l'instant de mesure, ainsi que par des déplacements prédits par le modèle prédictif en des instants précédent l'instant de mesure.
- d'une deuxième densité de probabilité conditionnelle $P \left(x={x}_{tg}\left|{x}_{c}^{s}, {x}_{p}^{s}\right.\right)$, conditionnée par la position ${x}_{c}^{s}$ de l'effecteur à l'instant de mesure et le déplacement prédit par le modèle prédictif ${x}_{p}^{s}$ à l'instant de mesure.

Selon une autre variante, l'estimation de la position de la cible, à chaque instant de mesure, est effectuée selon l'expression : $\hat{{x}_{tg}^{s}} = arg {max}_{x} \left[P\left(x={x}_{tg}\left|{x}_{c}^{1}…{x}_{c}^{s},{x}_{p}^{1}…{x}_{p}^{s}\right.\right)-{λ}_{1}\left‖\hat{{x}_{tg}^{s - 1}}-x\right‖-{λ}_{2}\left‖{x}_{c}^{s}-x\right‖\right]$

Où *λ*₁ et *λ*₂ sont des scalaires positifs, qui correspondent à des facteurs de régularisation.

Il s'agit ici de maximiser une grandeur établie à partir de la densité de probabilité de cible, diminuée :
- d'une première distance, entre la position de la cible $\hat{{x}_{tg}^{s - 1}}$ et chaque point x de l'espace, ladite première distance étant pondérée par un premier facteur de régularisation *λ*₁. Cela permet d'éviter que deux positions successives estimées de la cible soient trop éloignées l'une de l'autre ;
- et/ou d'une deuxième distance entre la position de l'effecteur ${x}_{c}^{s}$ à l'instant de mesure et chaque point x de l'espace, ladite deuxième distance étant pondérée par un deuxième facteur de régularisation *λ*₂. Cela permet d'éviter que la position estimée de la cible soit trop éloignée de la position de l'effecteur à l'instant de mesure. Cela permet de contenir la position de la cible dans un certain espace de travail, autour de la position courante.

L'expression (9) peut être mise en œuvre sans prendre en compte la première distance (*λ*₁ = 0) ou sans prendre en compte la deuxième distance (*λ*₂ = 0).

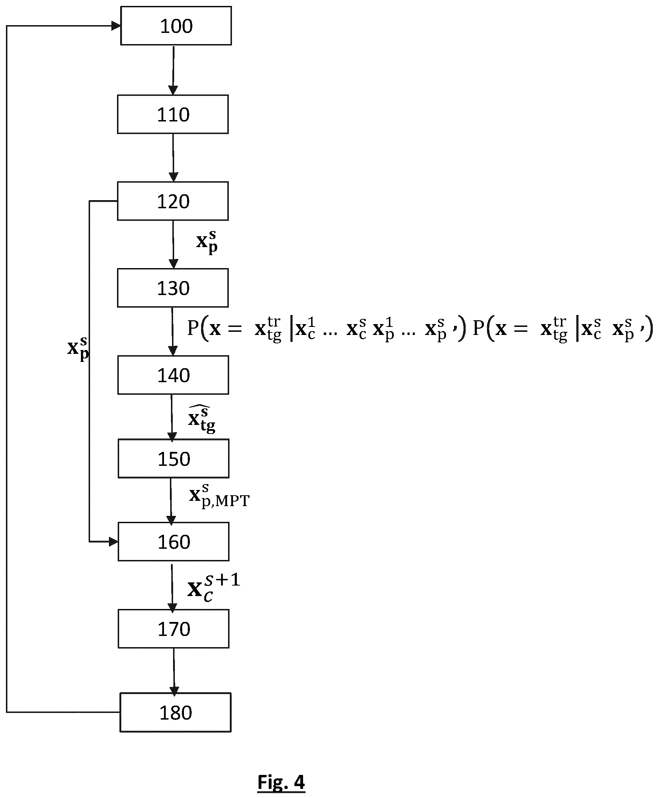

La figure 4 schématise les principales étapes d'un procédé mis en œuvre par une interface neuronale directe selon l'invention.

Etape 100 : prise en compte de signaux électrophysiologiques, émis par l'utilisateur et détectés par les capteurs 2 à un instant s ;
Etape 110 : à partir des signaux électrophysiologiques détectés, formation d'un signal d'entrée alimentant le modèle prédictif.

Etape 120 : définition, par le modèle prédictif, du déplacement ${x}_{p}^{s}$ à l'instant s, en fonction du signal d'entrée.

Etape 130 : détermination d'une densité de probabilité de cible, $P \left(x={x}_{tg}^{tr}\left|{x}_{c}^{1}…{x}_{c}^{s},{x}_{p}^{1}…{x}_{p}^{s}\right.\right) , P \left(x={x}_{tg}^{tr}\left|{x}_{c}^{s}, {x}_{p}^{s}\right.\right)$ en tout point x de l'espace dans lequel évolue l'effecteur, en fonction de la position de l'effecteur à l'instant s, du déplacement prédit à l'instant s, et éventuellement en fonction de la position de l'effecteur et du déplacement prédit à des instants précédents l'instant s : cf. expressions (3), (4) ou (8).

Etape 140 : estimation d'une position $\hat{{x}_{tg}^{s}}$ maximisant la densité de probabilité résultant de l'étape 130, ou d'une grandeur utilisant la densité de probabilité résultant de l'étape 130. Cf. expressions (5), (6) ou (9).

Etape 150 : à partir de $\hat{{x}_{tg}^{s}}$, calcul d'un déplacement vers la position estimée de la cible ${x}_{p , MPT}^{s}$

Etape 160 : à partir du déplacement ${x}_{p}^{s}$ défini par le modèle prédictif, résultant de l'étape 120, et du déplacement vers la position estimée de la cible, résultant de l'étape 150, détermination d'un déplacement corrigé ${x}_{c}^{s + 1} - {x}_{c}^{s}$: cf. expression (7)

Etape 170 : déplacement de l'effecteur en fonction du déplacement corrigé.

Etape 180 : incrémentation de l'indice temporel s et réitération des étapes 100 à 170 jusqu'à ce que la cible soit atteinte ou soit considérée comme atteinte, ou jusqu'à un arrêt volontaire de l'algorithme par l'utilisateur.

### Détermination de σ

L'écart type σ est préalablement déterminé au cours d'essais préalables. Au cours de ces essais, la position ${x}_{tg}^{tr}$ de la cible est connue. On peut établir une fonction de coût permettant d'estimer une précision angulaire, et déterminer la valeur de σ qui optimise la fonction de coût. La fonction de coût peut une fonction similarité cosinus moyenne *MCS* telle que : $MCS = \frac{1}{{N}_{ss}} {\sum }_{tr = 1}^{{N}_{ss}} \frac{1}{{s}_{tr}} {\sum }_{s = 1}^{{s}_{tr}} CosSim \left(\hat{{x}_{p}^{s}}, {x}_{d}^{s}\right)$ où
- *Nₛₛ* correspond à un nombre d'essais préalables réalisés ;
- *sₜᵣ* correspondant aux nombres d'incréments temporels au cours de chaque essai tr ;
- $CosSim \left({x}_{p}^{s}, {x}_{d}^{s}\right)$correspond à la fonction similarité cosinus, telle que : $CosSim \left({x}_{p}^{s}, {x}_{d}^{s}\right) = \frac{\hat{{x}_{p}^{s}} , {x}_{d}^{s} .}{\left‖\hat{{x}_{p}^{s}}\right‖ \left‖{x}_{d}^{s}\right‖}$
- $\hat{{x}_{p}^{s}} = \hat{{x}_{tg}^{s}} - {x}_{c}^{s} ;$
- ${x}_{d}^{s} = {x}_{tg}^{tr} - {x}_{c}^{s} . {x}_{d}^{s}$ correspond au déplacement désiré.

La valeur de σ est celle qui maximise la fonction de coût MCS.

La fonction de coût peut être une erreur moyenne de la position de la cible estimée par rapport à la position réelle de la cible, sur les derniers pas de temps, par exemple les 30 derniers incréments temporels. $MDE = \frac{1}{{N}_{ss}} {\sum }_{tr = 1}^{{N}_{ss}} \frac{1}{30} {\sum }_{s = {s}_{tr} - 29}^{{s}_{tr}} \left‖\hat{{x}_{tg}^{s}}-{x}_{tg}^{tr}\right‖$

*Nₛₛ* et *sₜᵣ* ayant été définis en lien avec (11).

La valeur de σ est celle qui minimise la fonction de coût MDE.

### Simulations

Des simulations ont été effectuées sur des jeux de données artificiels, dans lesquels la position de la cible était connue. Ainsi, à chaque instant, ${x}_{d}^{s}$ est connu. A partir de ${x}_{d}^{s}$, on a simulé le déplacement prédit par le modèle prédictif d'une interface en considérant que le déplacement prédit par le modèle prédictif ${x}_{p}^{s}$ est tel que : ${x}_{p}^{s} = {x}_{d}^{s} + {ε}^{s}$ où *ε^{s}* est un vecteur représentant un bruit.

On a pris en compte deux modèles de bruits différents : un premier modèle de bruit était un bruit gaussien indépendant et identiquement distribué (IID) de puissance η²=1.4. Un deuxième modèle de bruit était un bruit gaussien dépendant des incréments temporels précédents, tel que : ${ε}^{s} = {\sum }_{i = 1}^{10} {0.95}^{10 - i} ∗ {w}^{s - i}$

Où w suit une loi centrée IID de puissance η²=0.6.

La densité de probabilité de cible a été calculée en considérant σ² = 3 rad².

Une première série d'essai a été effectuée en considérant k = 1 dans (7). Au cours de ces essais, on a estimé une position de la cible à chaque incrément temporel. La position estimée de la cible n'a pas été utilisée dans le contrôle du déplacement de l'effecteur.

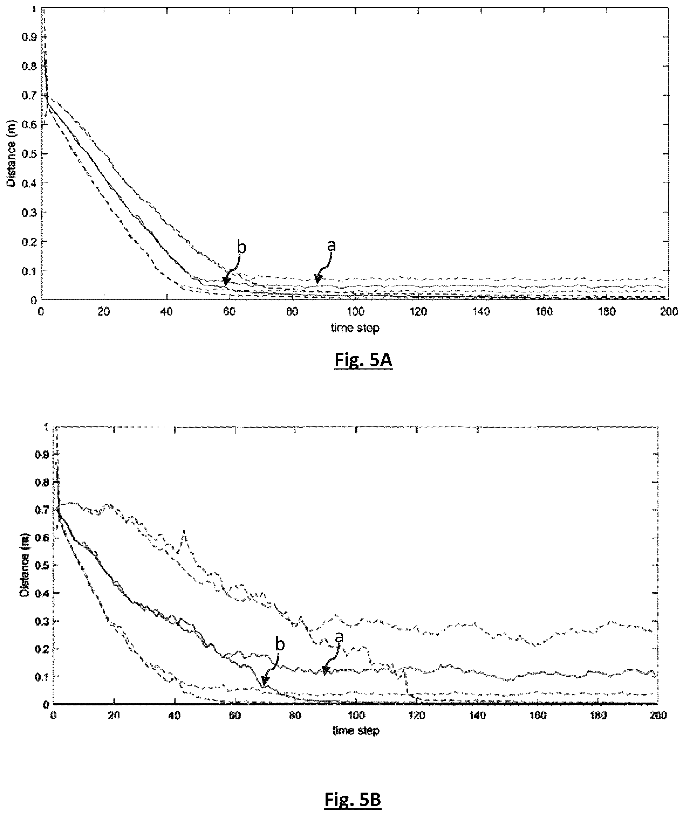

La figure 5A montre l'évolution, en fonction du temps, de la distance entre l'effecteur et la cible (courbe a) ainsi que la distance entre la cible estimée et la cible réelle (courbe b), en considérant le premier modèle de bruit. L'axe des abscisses correspond au temps et l'axe des ordonnées correspond aux distances étudiées.

La figure 5B montre l'évolution, en fonction du temps, de la distance entre l'effecteur et la cible (courbe a) ainsi que la distance entre la cible estimée et la cible réelle (courbe b), en considérant le deuxième modèle de bruit. L'axe des abscisses correspond au temps et l'axe des ordonnées correspond aux distances étudiées.

Sur les figures 5A et 5B, les courbes en tirets représentent les limites des intervalles de confiance.

Sur les deux figures, on observe que la position de la cible est stabilisée lorsque l'effecteur est suffisamment proche de la cible. *s*≥60 sur la figure 5A et *s*≥40 sur la figure 5B

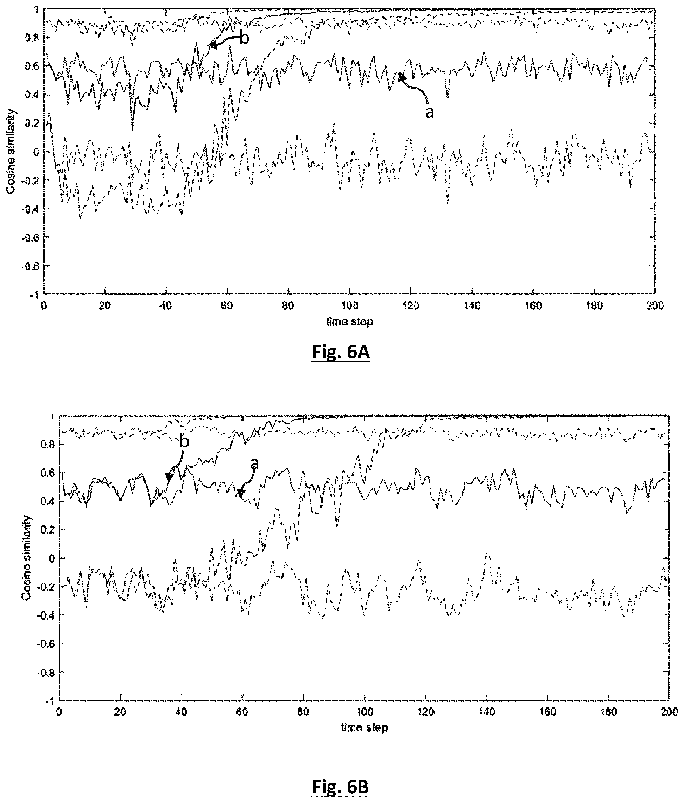

La figure 6A montre l'évolution, en fonction du temps, de la précision angulaire calculée en prenant en compte le modèle prédictif (courbe a) et l'estimation de la cible (courbe b), en considérant le premier modèle de bruit. L'axe des abscisses correspond au temps et l'axe des ordonnées correspond à la précision angulaire, exprimée sous la forme d'une similarité cosinus. La courbe a correspond à $CosSim \left({x}_{d}^{s}, {x}_{p}^{s}\right)$ et la courbe b correspond à $CosSim \left({x}_{d}^{s}, {x}_{p , MPT}^{s}\right)$. Cette courbe permet de comparer la performance de prédiction :
- du modèle prédictif seul (courbe a), ce qui revient à mettre en œuvre (7) avec k = 0 ;
- de l'estimation de la position de la cible, ce qui revient à mettre en œuvre (7) avec k = 1.

La figure 6B montre l'évolution, en fonction du temps, de la précision angulaire calculée en prenant en compte le modèle prédictif (courbe a) et l'estimation de la cible (courbe b), en considérant le deuxième modèle de bruit. L'axe des abscisses correspond au temps et l'axe des ordonnées correspond à la précision angulaire, exprimée sous la forme d'une similarité cosinus. De même que sur la figure 6A, la courbe a correspond à $CosSim \left({x}_{d}^{s}, {x}_{p}^{s}\right)$ et la courbe b correspond à $CosSim \left({x}_{d}^{s}, {x}_{p , MPT}^{s}\right)$.

On observe que la précision angulaire obtenue par l'estimation de la cible par la méthode proposée ici (courbes b) est meilleure par rapport à celle résultant du le modèle prédictif, et cela dès que l'effecteur est suffisamment proche de la cible : *s*≥60 sur la figure 6A (cf. figure 5A) et *s*≥40 sur la figure 6B (cf. figure 5B).

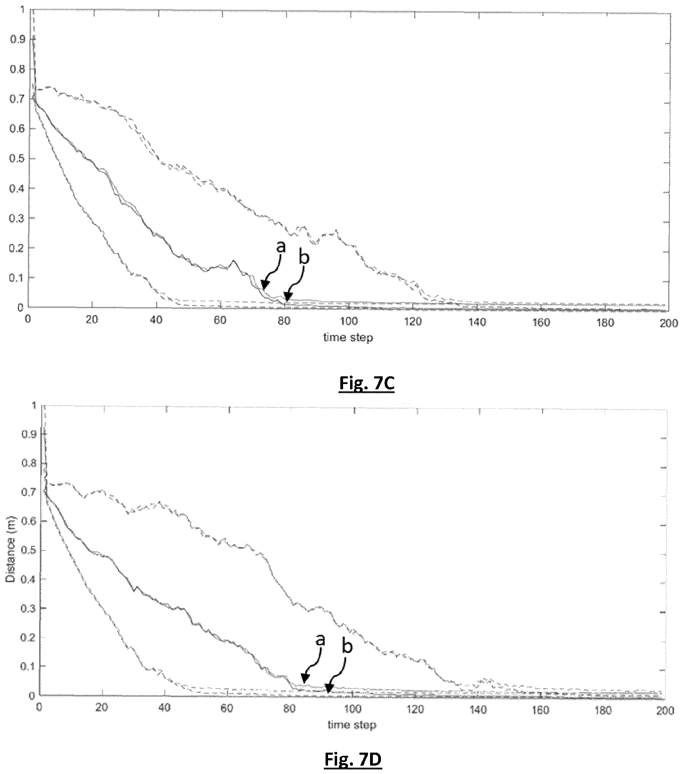

Les figures 7A à 7D représentent l'évolution, en fonction du temps, de la distance entre l'effecteur et la cible réelle (courbe a) ainsi que la distance entre la cible estimée et la cible réelle (courbe b), en considérant le second modèle de bruit. L'axe des abscisses correspond au temps et l'axe des ordonnées correspond aux distances étudiées. On a fait varier la valeur de k sur les différentes figures : figure 7A : k=0.9 ; figure 7B : k=0.7 ; figure 7C : k=0.5 ; figure 7D : k=0.

La diminution de k permet de mieux stabiliser la position de l'effecteur à proximité de la cible, au prix d'un certain retard au niveau de la convergence.

Les courbes des figures 7A à 7D peuvent être comparées à la figure 5B, cette dernière correspondant à un déplacement de l'effecteur sans prendre en compte la position estimée de la cible (k = 1).

### Mode de réalisation en trois dimensions

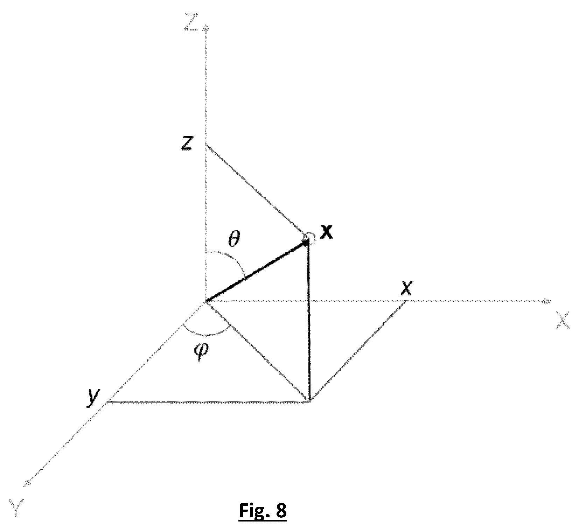

L'invention a été décrite en considérant un mouvement de l'effecteur en deux dimensions. Les mêmes principes peuvent s'appliquer en trois dimensions. La figure 8 montre les angles à prendre en compte, dans un référentiel à trois dimension, selon lequel chaque position x est définie par trois coordonnées (*x, y* z) $φ = arctan \left(\frac{y}{x}\right)$ $θ = arctan \left(\frac{\sqrt{{x}^{2} + {y}^{2}}}{z}\right)$

Les erreurs angulaires entre deux vecteurs x₁, de coordonnées (*x*₁, *y*₁, *z*₁) et x₂ de coordonnées (*x₂, y*₂, *z*₂) sont telles que : ${α}_{φ} \left({x}_{1}, {x}_{2}\right) = arctan \left(\frac{{y}_{1}}{{x}_{1}}\right) - arctan \left(\frac{{y}_{2}}{{x}_{2}}\right)$ et ${α}_{θ} \left({x}_{1}, {x}_{2}\right) = arctan \left(\frac{\sqrt{{{x}_{1}}^{2} + {{y}_{1}}^{2}}}{{z}_{1}}\right) - arctan \left(\frac{\sqrt{{{x}_{2}}^{2} + {{y}_{2}}^{2}}}{{z}_{2}}\right)$

En supposant que l'utilisateur cherche à réduire les erreurs angulaires à chaque pas temporel, et que les erreurs angulaires suivent des distributions gaussiennes indépendantes de même puissance, la densité de probabilité de cible, définie en 2D dans l'expression (3), devient : $P \left(x={x}_{tg}^{tr}\left|{x}_{c}^{s}, {x}_{p}^{s}\right.\right) = \frac{1}{2 π {σ}^{2}} {e}^{- \frac{{α}_{φ} {\left({x}_{c}^{s}-x,{x}_{p}^{s}\right)}^{2} - {α}_{θ} {\left({x}_{c}^{s}-x,{x}_{p}^{s}\right)}^{2}}{2 {σ}^{2}}}$

Les étapes 100 à 180, précédemment décrites, peuvent être mises en œuvre en 3 dimensions à partir de la densité de probabilité de cible définie dans (19).

## Revendications

1. Procédé de commande du déplacement, dans un espace, d'un effecteur par une interface neuronale directe, le déplacement tendant à rapprocher l'effecteur d'une position cible, le procédé comportant les étapes suivantes :
- a) acquisition de signaux électrophysiologiques produits dans le cortex d'un individu, à un instant de mesure, l'effecteur occupant une position, dans l'espace, audit instant de mesure ;
- b) traitement des signaux électrophysiologiques, pour former des données d'entrée ;
- c) traitement des données d'entrée, par un modèle prédictif mis en œuvre par l'interface neuronale, pour définir un déplacement de l'effecteur ( ${x}_{p}^{s}$) à l'instant de mesure;
- d) détermination d'un déplacement corrigé ( ${x}_{c}^{s + 1} - {x}_{c}^{s}$) de l'effecteur, à partir du déplacement défini par le modèle prédictif ( ${x}_{p}^{s}$) lors de l'étape c) ;
- e) commande du déplacement de l'effecteur dans l'espace, par l'interface neuronale directe, à partir du déplacement corrigé résultant de l'étape d) ;
- f) réitération des étapes a) à e), en incrémentant l'instant de mesure (s), jusqu'à l'atteinte d'un critère d'arrêt des itérations ;
le procédé comportant, lors de chaque étape d) suivant au moins une première itération des étapes a) à e), :
- di) estimation de la position cible ( $\hat{{x}_{tg}^{s}}$), à l'instant de mesure, à partir d'au moins :
• une position de l'effecteur ( ${x}_{c}^{s}$) à l'instant de mesure, ainsi qu'à au moins un instant précédent l'instant de mesure ;
• et un déplacement défini par le modèle prédictif ( ${x}_{p}^{s}$) à l'instant de mesure ainsi qu'à au moins l'instant précédent l'instant de mesure ;
- dii) à partir de la position cible estimée lors de la sous-étape di), détermination d'un déplacement vers la cible estimée ( ${x}_{p , MPT}^{s}$);
- diii) prise en compte du déplacement vers la cible estimée ( ${x}_{p , MPT}^{s}$), résultant de la sous-étape dii), pour établir le déplacement corrigé ( ${x}_{c}^{s + 1} - {x}_{c}^{s}$) à l'instant de mesure.

2. Procédé selon la revendication 1, dans lequel la sous-étape di) comporte :
- calcul d'une densité de probabilité de cible, correspondant à une densité de probabilité que chaque point de l'espace corresponde à la position cible ;
- détermination du point de l'espace maximisant la densité de probabilité de cible, ou une grandeur comportant la densité de probabilité de cible, le point ainsi déterminé correspondant à la position cible estimée.

3. Procédé selon la revendication 2, dans lequel le calcul de la densité de probabilité de cible prend en compte :
• plusieurs positions de l'effecteur ( ${x}_{c}^{1} … {x}_{c}^{s}$) à des instants précédent l'instant de mesure ;
• plusieurs déplacements définis par le modèle prédictif ( ${x}_{p}^{1} … {x}_{p}^{s}$) à des instants précédents l'instant de mesure.

4. Procédé selon la revendication 3, dans lequel le calcul de la densité de probabilité de cible est établi à partir d'une combinaison :
- d'une première densité de probabilité conditionnelle ( $P \left(x={x}_{tg}\left|{x}_{c}^{1}…{x}_{c}^{s - 1},{x}_{p}^{1}…{x}_{p}^{s - 1}\right.\right)$ ), conditionnée par
• des positions de l'effecteur à des instants précédent l'instant de mesure;
• des déplacements définis par le modèle prédictif à des instants précédent l'instant de mesure ;
- et d'une deuxième densité de probabilité conditionnelle, $P \left(x={x}_{tg}\left|{x}_{c}^{s}, {x}_{p}^{s}\right.\right)$ conditionnée par :
• la position de l'effecteur à l'instant de mesure ;
• le déplacement défini par le modèle prédictif à l'instant de mesure.

5. Procédé selon la revendication 4, dans lequel la première densité de probabilité conditionnelle est pondérée par un facteur d'oubli (*µ*).

6. Procédé selon l'une quelconque des revendications 2 à 5, dans lequel la sous-étape di) comporte une détermination du point maximisant une grandeur comportant la densité de probabilité cible, la grandeur correspondant à la densité de probabilité cible diminuée :
• d'une première distance ( $\left‖\hat{{x}_{tg}^{s - 1}}-x\right‖$), pondérée par un premier facteur de régularisation (*λ*₁), entre chaque point et la position cible estimée à un instant de mesure précédent ;
• et/ou d'une deuxième distance ( $\left‖{x}_{c}^{s}-x\right‖$), pondérée par un deuxième facteur de régularisation (*λ*₂), entre chaque point et la position de l'effecteur à l'instant de mesure.

7. Procédé selon l'une quelconque des revendications précédentes, dans lequel lors de la sous-étape dii), le déplacement vers la cible estimée est établi de façon que l'interface déplace l'effecteur de la position à l'instant de mesure vers la position cible estimée à l'instant de mesure.

8. Procédé selon l'une quelconque des revendications précédentes, dans lequel lors de la sous-étape diii), le déplacement corrigé est une combinaison du déplacement défini par le modèle prédictif lors de l'étape b) et du déplacement vers la cible estimée résultant de la sous-étape dii).

9. Procédé selon la revendication 8, dans lequel la combinaison met en œuvre
- un premier facteur de pondération (k), appliqué au déplacement défini par le modèle prédictif ;
- un deuxième facteur de pondération (1-k), appliqué au déplacement vers la cible estimée.

10. Interface neuronale directe (1), comportant :
- des capteurs (2), aptes à acquérir des signaux électrophysiologiques représentatifs d'une activité corticale ;
- un effecteur (5), configuré pour être actionné par un signal de commande généré par l'interface neuronale directe ;
- un processeur programmé pour déterminer un déplacement de l'effecteur en fonction de données d'entrée résultant des signaux électrophysiologiques détectés ;
le processeur (3) étant configuré pour mettre en œuvre les étapes c) à f) d'un procédé objet de l'une quelconque des revendications précédentes, en différents instants de mesure.

## Patentansprüche

1. Verfahren zum Steuern der Bewegung, in einem Raum, eines Effektors durch eine direkte neuronale Schnittstelle, wobei die Bewegung dazu tendiert, den Effektor einer Zielposition zu nähern, wobei das Verfahren die folgenden Schritte umfasst:
- a) Erfassen von elektrophysiologischen Signalen, die im Kortex einer Person zu einem Messzeitpunkt erzeugt werden, wobei der Effektor eine Position, in dem Raum, zu dem Messzeitpunkt einnimmt;
- b) Verarbeiten der elektrophysiologischen Signale, um Eingangsdaten zu bilden;
- c) Verarbeiten der Eingangsdaten durch ein von der neuronalen Schnittstelle implementiertes prädiktives Modell, um eine Bewegung des Effektors ( ${x}_{p}^{s}$) zu dem Messzeitpunkt zu definieren;
- d) Bestimmen einer korrigierten Bewegung ( ${x}_{c}^{s + 1} - {x}_{c}^{s}$) des Effektors ausgehend von der durch das prädiktive Modell ( ${x}_{p}^{s}$) beim Schritt c) definierten Bewegung;
- e) Steuern der Bewegung des Effektors in dem Raum durch die direkte neuronale Schnittstelle ausgehend von der aus dem Schritt d) resultierenden korrigierten Bewegung;
- f) Wiederholen der Schritte a) bis e) unter Inkrementierung des Messzeitpunkts (s) bis zum Erreichen eines Stoppkriteriums der Iterationen;
wobei das Verfahren bei jedem Schritt d) nach mindestens einer ersten Iteration der Schritte a) bis e) Folgendes umfasst:
- di) Schätzen der Zielposition ( $\hat{{x}_{tg}^{s}}$) zu dem Messzeitpunkt ausgehend von mindestens:
• einer Position des Effektors ( ${x}_{c}^{s}$) zu dem Messzeitpunkt sowie zu mindestens einem Zeitpunkt vor dem Messzeitpunkt;
• und einer durch das prädiktive Modell ( ${x}_{p}^{s}$) zu dem Messzeitpunkt sowie zu mindestens dem Zeitpunkt vor dem Messzeitpunkt definierten Bewegung;
- dii) ausgehend von der beim Teilschritt di) geschätzten Zielposition, Bestimmen einer Bewegung zu dem geschätzten Ziel ( ${x}_{p , MPT}^{s}$) hin;
- diii) Berücksichtigen der aus dem Teilschritt dii) resultierenden Bewegung zu dem geschätzten Ziel ( ${x}_{p , MPT}^{s}$) hin, um die korrigierte Bewegung ( ${x}_{c}^{s + 1} - {x}_{c}^{s}$) zu dem Messzeitpunkt zu ermitteln.

2. Verfahren nach Anspruch 1, wobei der Teilschritt di) umfasst:
- Berechnen einer Zielwahrscheinlichkeitsdichte, entsprechend einer Wahrscheinlichkeitsdichte, dass jeder Punkt des Raums der Zielposition entspricht;
- Bestimmen des Punktes des Raums, der die Zielwahrscheinlichkeitsdichte oder eine die Zielwahrscheinlichkeitsdichte umfassende Größe maximiert, wobei der so bestimmte Punkt der geschätzten Zielposition entspricht.

3. Verfahren nach Anspruch 2, wobei die Berechnung der Zielwahrscheinlichkeitsdichte Folgendes berücksichtigt:
• mehrere Positionen des Effektors ( ${x}_{c}^{1} … {x}_{c}^{s}$) zu Zeitpunkten vor dem Messzeitpunkt;
• mehrere durch das prädiktive Modell ( ${x}_{p}^{1} … {x}_{p}^{s}$) zu Zeitpunkten vor dem Messzeitpunkt definierte Bewegungen.

4. Verfahren nach Anspruch 3, wobei die Berechnung der Zielwahrscheinlichkeitsdichte ermittelt wird ausgehend von einer Kombination:
- aus einer ersten bedingten Wahrscheinlichkeitsdichte ( $P \left(x={x}_{tg}\left|{x}_{c}^{1}…{x}_{c}^{s - 1},{x}_{p}^{1}…{x}_{p}^{s - 1}\right.\right)$), die bedingt ist durch
• Positionen des Effektors zu Zeitpunkten vor dem Messzeitpunkt;
• durch das prädiktive Modell zu Zeitpunkten vor dem Messzeitpunkt definierte Bewegungen;
- und aus einer zweiten bedingten Wahrscheinlichkeitsdichte $P \left(x={x}_{tg}\left|{x}_{c}^{s}, {x}_{p}^{s}\right.\right)$, die bedingt ist durch
• die Position des Effektors zu dem Messzeitpunkt;
• die durch das prädiktive Modell zu dem Messzeitpunkt definierte Bewegung.

5. Verfahren nach Anspruch 4, wobei die erste bedingte Wahrscheinlichkeitsdichte durch einen Vergessensfaktor (µ) gewichtet wird.

6. Verfahren nach einem der Ansprüche 2 bis 5, wobei der Teilschritt di) ein Bestimmen des Punktes umfasst, der eine die Zielwahrscheinlichkeitsdichte umfassende Größe maximiert, wobei die Größe der Zielwahrscheinlichkeitsdichte entspricht abzüglich von:
• einer ersten Distanz ( $\left‖\hat{{x}_{tg}^{s - 1}}-x\right‖$), die durch einen ersten Regularisierungsfaktor (λ₁) gewichtet wird, zwischen jedem Punkt und der zu einem früheren Messzeitpunkt geschätzten Zielposition;
• und/oder einer zweiten Distanz ( $\left‖{x}_{c}^{s}-x\right‖$), die durch einen zweiten Regularisierungsfaktor (λ₂) gewichtet wird, zwischen jedem Punkt und der Position des Effektors zu dem Messzeitpunkt.

7. Verfahren nach einem der vorhergehenden Ansprüche, wobei bei dem Teilschritt dii) die Bewegung zu dem geschätzten Ziel hin so ermittelt wird, dass die Schnittstelle den Effektor von der Position zu dem Messzeitpunkt zu der zu dem Messzeitpunkt geschätzten Zielposition bewegt.

8. Verfahren nach einem der vorhergehenden Ansprüche, wobei bei dem Teilschritt diii) die korrigierte Bewegung eine Kombination aus der durch das prädiktive Modell beim Schritt b) definierten Bewegung und aus der aus dem Teilschritt dii) resultierenden Bewegung zu dem geschätzten Ziel hin ist.

9. Verfahren nach Anspruch 8, wobei die Kombination Folgendes implementiert:
- einen ersten Gewichtungsfaktor (k), der auf die durch das prädiktive Modell definierte Bewegung angewendet wird;
- einen zweiten Gewichtungsfaktor (1-k), der auf die Bewegung zu dem geschätzten Ziel hin angewendet wird.

10. Direkte neuronale Schnittstelle (1), umfassend:
- Sensoren (2), die geeignet sind, elektrophysiologische Signale zu erfassen, die für eine kortikale Aktivität repräsentativ sind;
- einen Effektor (5), der dazu ausgestaltet ist, durch ein von der direkten neuronalen Schnittstelle erzeugtes Steuersignal in Gang gesetzt zu werden;
- einen Prozessor, der dazu programmiert ist, eine Bewegung des Effektors in Abhängigkeit von Eingangsdaten zu bestimmen, die aus den detektierten elektrophysiologischen Signalen resultieren;
wobei der Prozessor (3) dazu ausgestaltet ist, die Schritte c) bis f) eines Verfahrens, das Gegenstand eines der vorhergehenden Ansprüche ist, zu verschiedenen Messzeitpunkten zu implementieren.

## Claims

1. Method for controlling the movement, through a space, of an effector via a brain-computer interface, the movement tending to bring the effector closer to a target position, the method comprising the following steps:
- a) acquiring electrophysiological signals produced in the cortex of an individual, at a measurement time, the effector occupying a position, in the space, at said measurement time;
- b) processing the electrophysiological signals, to form input data;
- c) processing the input data, by means of a predictive model implemented by the brain-computer interface, to define an effector movement ( ${x}_{p}^{s}$) at the measurement time;
- d) determining a corrected effector movement ( ${x}_{c}^{s + 1} - {x}_{c}^{s}$), based on the movement defined by the predictive model ( ${x}_{p}^{s}$) in step c);
- e) controlling the movement of the effector through the space, by means of the brain-computer interface, based on the corrected movement resulting from step d);
- f) incrementing the measurement time (s) and reiterating steps a) to e) until a criterion for ending the iterations is met;
the method comprising, in each step d) following at least a first iteration of steps a) to e):
- di) estimating the target position ( ${\hat{x}}_{tg}^{s}$), at the measurement time, based on at least:
• an effector position ( ${x}_{c}^{s}$) at the measurement time and at least one time preceding the measurement time;
• and a movement defined by the predictive model ( ${x}_{p}^{s}$) at the measurement time and at least at the time preceding the measurement time;
- dii) based on the target position estimated in sub-step di), determining a movement towards the estimated target ( ${x}_{p , MPT}^{s}$);
- diii) taking into account the movement towards the estimated target, resulting from sub-step dii), to establish the corrected movement ( ${x}_{c}^{s + 1} - {x}_{c}^{s}$) at the measurement time.

2. Method according to Claim 1, wherein sub-step di) comprises:
- computing a target probability density, corresponding to a probability density reflecting the probability that each point in the space corresponds to the target position;
- determining the point in the space maximizing the target probability density, or a quantity comprising the target probability density, the point thus determined corresponding to the estimated target position.

3. Method according to Claim 2, wherein computation of the target probability density takes into account:
• a plurality of effector positions ( ${x}_{c}^{1} … {x}_{c}^{s}$) at times preceding the measurement time;
• a plurality of movements ( ${x}_{p}^{1} … {x}_{p}^{s}$) defined by the predictive model at times preceding the measurement time.

4. Method according to Claim 3, wherein the target probability density is computed based on a combination:
- of a first conditional probability density ( $P \left(x={x}_{tg}\left|{x}_{c}^{1}… {x}_{c}^{s - 1},{x}_{p}^{1}… {x}_{p}^{s - 1}\right.\right)$) dependent on:
• effector positions at times preceding the measurement time;
• movements defined by the predictive model at times preceding the measurement time;
- and of a second conditional probability density $P \left(x={x}_{tg}\left|{x}_{c}^{s}, {x}_{p}^{s}\right.\right)$ dependent on:
• the effector position at the measurement time;
• the movement defined by the predictive model at the measurement time.

5. Method according to Claim 4, wherein the first conditional probability density is weighted by a forgetting factor (*µ*).

6. Method according to any one of Claims 2 to Claim 5, wherein sub-step di) comprises determining the point maximizing a quantity comprising the target probability density, the quantity corresponding to the target probability density decreased:
• by a first distance ( $\left‖\hat{{x}_{tg}^{s - 1}}-x\right‖$), weighted by a first regularization factor (*λ*₁), between each point and the target position estimated at a preceding measurement time;
• and/or by a second distance ( $\left‖{x}_{c}^{s}-x\right‖$), weighted by a second regularization factor (*λ*₂), between each point and the effector position at the measurement time.

7. Method according to any one of the preceding claims, wherein, in sub-step dii), the movement towards the estimated target is established so that the interface moves the effector from the position at the measurement time to the target position estimated at the measurement time.

8. Method according to any one of the preceding claims, wherein, in sub-step diii), the corrected movement is a combination of the movement defined by the predictive model in step b) and of the movement towards the estimated target resulting from sub-step dii).

9. Method according to Claim 8, wherein the combination employs:
- a first weighting factor (*k*), applied to the movement defined by the predictive model;
- a second weighting factor (1-*k*), applied to the movement towards the estimated target.

10. Brain-computer interface (1), comprising:
- sensors (2), able to acquire electrophysiological signals representative of a cortical activity;
- an effector (5), configured to be actuated by a control signal generated by the brain-computer interface;
- a processor programmed to determine an effector movement depending on input data resulting from the detected electrophysiological signals;
the processor (3) being configured to implement steps c) to f) of a method according to any one of the preceding claims, at various measurement times.
